# EUROPEAN PATENT SPECIFICATION

(11) **EP 2 803 363 B1**
(45) Date of publication and mention of the grant of the patent: **05.04.2017**
(21) Application number: 13167550.6
(22) Date of filing: 13.05.2013
(51) Int. Cl.: A61K 39/00, A61P 37/06, A61P 35/00, A61P 35/02

(54) **The antigen CYBA-72Y/B15 and uses thereof**
Das Antigen CYBA-72Y/B15 und Verwendungen davon
Antigène CYBA-72Y/B15 et leurs utilisations

(43) Date of publication of application: 19.11.2014
(73) Proprietor: Universitätsmedizin der Johannes Gutenberg-Universität Mainz, 55131 Mainz (DE)
(72) Inventor: Wölfel, Catherine, 55128 Mainz (DE); Domning, Sabine, SW16 2DZ London (GB); Lennerz, Volker, 55270 Ober-Olm (DE); Wölfel, Thomas, 55128 Mainz (DE); Distler, Eva, 64289 Darmstadt (DE); Herr, Wolfgang, 55131 Mainz (DE)
(74) Representative: Krauss, Jan

(56) References cited:
- WO-A1-02/064798
- DATABASE UniProt [Online] 18 April 2012 (2012-04-18), "SubName: Full=Cytochrome b-245 light chain; Flags: Fragment;", XP002714313, retrieved from EBI accession no. UNIPROT:H3BPX1 Database accession no. H3BPX1
- DATABASE UniParc [Online] 13 September 2006 (2006-09-13), XP002714314, retrieved from Uniprot Database accession no. UPI0000E02E96
- FENG X ET AL: "Targeting minor histocompatibility antigens in graft versus tumor or graft versus leukemia responses", TRENDS IN IMMUNOLOGY, vol. 29, no. 12, 1 December 2008 (2008-12-01), pages 624-632, XP025681420, ELSEVIER LTD. TRENDS JOURNALS, GB ISSN: 1471-4906, DOI: 10.1016/J.IT.2008.09.004 [retrieved on 2008-10-25]
- AKATSUKA YOSHIKI ET AL: "Minor histocompatibility antigens as targets for immunotherapy using allogeneic immune reactions.", CANCER SCIENCE, vol. 98, no. 8, August 2007 (2007-08), pages 1139-1146, XP002714315, ISSN: 1347-9032

### SEQUENCE LISTING

<110> Universitätsmedizin der Johannes Gutenberg-Universität Mainz
<120> The Antigen CYBA-72YB15 and uses thereof
<130> U30496EP
<160> 3
<170> Patent In version 3.5
<210> 1
   <211> 9
   <212> PRT
   <213> homo sapiens
<400> 1
   Gly Gln Lys Tyr Met Thr Ala Val Val
   1               5
<210> 2
   <211> 195
   <212> PRT
   <213> homo sapiens
<400> 2
<210> 3
   <211> 10
   <212> PRT
   <213> homo sapiens
<400> 3
   Trp Gly Gln Lys Tyr Met Thr Ala Val Val
   1               5                   10

## Description

The present invention relates to a novel minor histocompatibility antigen (mHAg) based on CYBA-72Y/B15 that elicits a CD8-positive T-cell response, as well as uses thereof in therapy and diagnosis. The invention is defined by the appended set of claims.

### DESCRIPTION

T lymphocytes recognize short peptides that are processed from proteins of all subcellular localizations, and are presented on the cellular surface in the context of HLA molecules. They are essential mediators of antiviral and antitumoral immune responses, as well as of reactions leading to tissue rejection. The allogeneic hematopoietic stem cell transplantation (allo-HSCT) still remains a curative treatment of many severe diseases, especially hematopoietic malignancies, such as leukemia. In allo-HSCT, T lymphocytes mediate both the desired graft-versus-leukemia (GvL-, or graft-versus-tumor, GvT)-effect but also the undesired graft-versus-host disease (GvHD) that is associated with high morbidity and also mortality.

Beneficial GvL/GvT and detrimental GvHD effects frequently co-occur after HSCT and seem to be functionally connected. For example, patients with acute or chronic GvHD after allo-HSCT have a lower risk of cancer relapse. This is due to specific responses of engrafted donor T lymphocytes to antigens expressed by thepatient's malignant cells. HSCT from HLA-matched unrelated donors results in a lower relapse rates than HSCT from identical twins, underlining the assumption that genetic differences between patients and donors can be targeted by this type of immunotherapy. GvL/GvT effects become most effective in transplantation settings that employ less intensive immuno-suppressive regimens. Due to these insights allo-HSCT is a well-established immunotherapy in this day and age.

The successful donor search is one of the most important factors deciding about the success of allo-HSCT. Despite the improved matching of donor-recipient pairs that became possible after implementation of high-resolution HLA-typing methods and a further optimization of allo-HSCT procedures, the outcome is still compromised by frequent complications such as GvHD, engraftment failure (lack or loss of engraftment), and disease relapse (H. Greinix, "Introduction," in Graft -Versus-Host Disease, H. T. Greinix, Ed., pp. 14-15, Unimed Verlag AG, 2008). Long-term survival after allo-HSCT is observed in 40-70% of the patients. Failures are mainly due to infectious complications and GvHD (30-40% each), organ toxicity of chemotherapy (20%), and relapse (20-30%).

In completely HLA-matched allo-HSCT situations, both GvL/GvT effects as well as GvHD, are primarily caused by T cells recognizing minor histocompatibility antigens (mHAgs). mHAg-encoding alleles differ between donor and recipient in missense single nucleotide polymorphisms (SNPs) in their coding regions. The changes of amino acid sequences caused by these SNPs lead to the production of immunogenic peptides and, thus, cause the immunogenicity of mHAgs.

When polymorphic peptides consisting of 8-12 amino acids bind to HLA class I molecules when longer peptides bindi to HLA class II molecules they are presented on the cell surface and can be recognized by T lymphocytes. Resulting tissue damage in vivo correlates with the expression distribution of the mHAgs: For example, responses of donor-derived transplanted T cells against mHAgs preferentially expressed on hematopoietic cells of the recipient can at the same time eliminate the recipient's hematopoietic system andleukemic cells.

So far, about 30 mHAgs have been identified in humans (Akatsuka et al., 2007; Feng et al., 2008). The identification of new mHAgs is advantageous, since knowledge thereof improves the selection of donors. In particular mHAgs with strictly tissue-specific expression can be used as therapeutic targets. IN contrast, ubiquitously expressed mHAgs are prone to induce GvHD. In one recently published example, UTA2-1-specific CTL effectively lysed mHAg-mismatched hematopoietic cells, including patients' myeloma cells, without affecting non-hematopoietic cells. As the authors summarized and concluded, "with the capacity to induce relevant immunotherapeutic CTLs, (with) its HLA-A*02 restriction and equally balanced phenotype frequency, UTA2-1 is a highly valuable mHAg to facilitate clinical application of mHAg-based immunotherapy" (Oostvogels R, et al. Towards effective and safe immunotherapy after allogeneic stem cell transplantation: identification of hematopoietic-specific minor histocompatibility antigen UTA2-1. Leukemia. 2013 Mar; 27(3):642-9).

US 6,830,883 describes a (poly)peptide comprising a T-cell epitope obtainable from the minor histocompatibility antigen HA-1, comprising the sequence VLXDDLLEA. Further disclosed is HA-1 typing, detection of genetic aberrances, and the like.

US 6,521,598 and US 7,008,922 disclose amino acid sequences SPSVDKARAEL and FIDSYICQV from the minor histocompatibility antigen H-Y. The peptides induce tolerance for transplantations when administered to H-Y-negative recipients.

WO 02/064798 discloses method for the diagnosis of cancer, or a predisposition thereto, in a patient determining the level of expression alpha polypeptide (CYBA). The document also discloses a CYBA antibody. Feng X et 1., 2008 discloses minor histocompatibility antigens and their use as immunotherapeutic targets.

In view of the above, it is an objective of the present invention to provide a novel mHAg which allows for improved diagnostic and therapeutic approaches in the context of allo-HSCT. Other objectives and advantages of the present invention will become apparent from studying the following more detailed description of the invention.

In a first aspect of the present invention, the above objective is solved by providing a peptide, consisting of an amino acid sequence according to SEQ ID No. 1 (GQKYMTAVV), wherein said peptide is capable of inducing a T-cell response according to the appended claim 1. Further embodiments of the invention are described in the.

The cytochrome b-245 alpha-polypeptide (CYBA, or p22phox) encodes for the membrane-bound small subunit of the NADPH-oxidase complexes 1-4 (NOX 1-4) that form reactive oxygen radicals (ROS). In addition to a function in the removal of pathogens, the ROS play a role in signaling and differentiation processes as well as in the regulation of gene expression (Bederd and Krause, 2007, Brar *et al.,* 2002). In agreement with their broad spectrum of functions, the NADPH oxidases are found to be expressed in a multitude of cell types (e.g. phagocytes, endothelial and muscle cells, fibroblasts).

Preferred is a peptide according to the present invention, wherein said peptide is capable of binding to an HLA class I molecule.

The inventors identified the minor histocompatibility antigen CYBA-72Y/B15 as target antigen of AML (acute myeloid leukemia)-reactive T lymphocytes . The antigen is encoded by the SNP rs4673 (alleles *CYBA*-*242C*/*T*) leading to a histidin(H)-to-tyrosin(Y) exchange at amino acid position 72. So far, only CYBA-72Y (encoded by *CYBA-242T*) was found to be immunogenic and to be recognized by T cells from individuals homozygous for the alleles encoding CYBA-72H (encoded by *CYBA-242C*). The target peptide sequence of CYBA-72Y/B15-reactive T cells was GQKYMTAW containing the polymorphic residue on its 4^{th} position. The allele *CYBA-242T* was found in 56 % of all tested healthy individuals and leukemia patients (in a cohort of n=481). The peptide-specific T lymphocytes efficiently recognized monocytes and dendritic cells, B cells to a lesser extent, but not granulocytes and not T lymphocytes. The T cells against the antigen of the invention also effectively lysed malignant cells. Thus, the present minor-histocompatibility antigen appears suitable to mediate the elimination of the hematopoiesis of the AML patients, including AML cells.

CYBA-72Y/B 15 represents a new mHAg that is naturally processed and is presented as HLA-bound peptide antigen on the surfaces of leukemic and other malignoma cells. Due to the preferred expression of CYBA in hematopoietic cells (http://www.proteinatlas.org/search/CYBA), CYBA-72Y has the potential to cause an GvL-effect following allo-HCST with elimination of the recipients'hematopoiesis, and to prevent a leukemia relapse. Since CYBA is also involved in the regulation of growth, metastasis and angiogenesis of malignant cells, a stable expression of CYBA similar to the expression of oncogenes can be assumed.

In order to improve CYBA-72Y/B15-specific immune responses, donors can be immunized before and recipients can be immunized after an allo-HSCT using different antigen formats . Furthermore, the GvL-effect can be enhanced by adoptive transfer of CYBA-72Y/B15-specific T cells or Tcell receptors. Due to the broad expression of CYBA in different malignomas, CYBA-Y72/B15-specific immune therapy can be considered in various tumor entities in combination with allo-HSCT.

In case future investigations point to an increased risk for GvHD after allo-HSCT in immunogenic CYBA-72-mismatch situations, the knowledge of the present mHAg will allow for the identification and depletion of antigen-specific T-cells in the transplant or in donor lymphocyte infusions (DLI), respectively. In addition, the knowledge of the mHAg will allow for the improved identification of suitable donors, in particular in allo-HSCT.

Further disclosed is a polypeptide which comprises at least 9, preferably 15, 20, 50, and most preferably 100 contiguous amino acids from the amino acid sequence of CYBA-72Y/B15 (SEQ ID No. 2), wherein said polypeptide is capable of inducing a T-cell response and/or binding a cognate T-cell receptor.

In the context of the present invention the terms "protein" or "polypeptide" are used interchangeably and denote a polymer composed of amino acid monomers joined by peptide bonds. A "peptide bond" is a covalent bond between two amino acids in which the α-amino group of one amino acid is bonded to the α-carboxyl group of the other amino acid. All amino acid or polypeptide sequences, unless otherwise designated, are written from the amino terminus (N-terminus) to the carboxy terminus (C-terminus). The terms "protein", "protein fragment" and "polypeptide" refer to a molecular chain of amino acids, and do not refer to a specific length of the product and if required can be modified *in vivo* or *in vitro,* for example by glycosylation, amidation, carboxylation or phosphorylation. Thus, *inter alia* peptides, oligopeptides and proteins are included within the definition of polypeptide.

Of course, functional derivatives and fragments of the polypeptide, summarized under the term "protein fragment", are also disclosed. Functional derivatives are meant to include polypeptides which differ in one or more amino acids in the overall sequence, which have deletions, substitutions, inversions, insertions or additions. Amino acid substitutions which can be expected not to essentially alter biological and immunological activities have been described. Amino acid replacements between related amino acids or replacements which have occurred frequently in evolution are *inter alia* Ser/Ala, Ser/Gly, Asp/Gly, Asp/Asn, Ile/Val.

In addition, the term "functional derivatives" of these polypeptides also implies the addition of salts of the polypeptides, amides of the polypeptides and specifically the C-terminal amides, esters and specifically the C-terminal esters and N-acyl derivatives specifically N-terminal acyl derivatives and N-acetyl derivatives.

In accordance with the present disclosure, the polypeptide is a protein, protein fragment or polypeptide comprising an amino acid sequence with at least 50%, 60%, 70%, 80%, 90%, 95%, most preferably 99% and even more preferably 100% sequence identity to the amino acid sequence of the protein CYBA-72Y/B15, specifically to the amino acid sequence as shown in SEQ ID No. 2. In the present invention, the term "identity" is determined by comparing two sequences aligned under optimal conditions over the sequences to be compared. The sequences to be compared herein may have an addition or deletion (for example, gap and the like) in the optimum alignment of the two sequences. Such a sequence homology (or identity) can be calculated by creating an alignment using, for example, the ClustalW algorithm (Nucleic Acid Res., 22(22): 4673 4680 (1994)).

The peptides according to the invention can be produced either synthetically or by recombinant DNA technology. Methods for producing synthetic polypeptides are well known in the art.

In preferred embodiments of the invention, the polypeptide consists of the amino acid sequence shown in SEQ ID No. 1.

In preferred embodiments, protein fragments of the herein described antigens are immunogenic fragments that still have the ability to induce an immunologic response, and which may also be used in order to generate antibodies that are specific against the fragment, such as monoclonal antibodies or specific fragments thereof, such as Fab or scFv. The peptide according to the invention can be used to generate and develop specific antibodies against MHC/peptide complexes. These can be used for therapy, targeting toxins or radioactive substances to the diseased tissue. Another use of these antibodies can be targeting radionuclides to the diseased tissue for imaging purposes such as PET. This use can help to detect small metastases or to determine the size and precise localization of diseased tissues.

Also disclosed is a fusion protein composed of the aforementioned polypeptide and of a second protein or polypeptide. Such fusion proteins are suitable for use as a diagnostic or therapeutic or prophylactic agent or generally for a detection and/or manipulation of T cells that recognize CYBA. For example, fusion proteins could be envisioned that consist of a carrier protein such as, for example, HSA, collagen or other proteins and the polypeptide of the invention.

Monoclonal antibodies may be prepared using hybridoma methods. In a hybridoma method, a mouse or other appropriate host animal is typically immunized with an immunizing agent to elicit lymphocytes that produce or are capable of producing antibodies that will specifically bind to the immunizing agent. Alternatively, the lymphocytes may be immunized *in vitro.*

The monoclonal antibodies may also be made by recombinant DNA methods, such as those described in U.S. Pat. No.4,816,567. DNA encoding the monoclonal antibodies of the invention can be readily isolated and sequenced using conventional procedures (e.g., by using oligonucleotide probes that are capable of binding specifically to genes encoding the heavy and light chains of murine antibodies).

*In vitro* methods are also suitable for preparing monovalent antibodies. Digestion of antibodies to produce fragments thereof, particularly, Fab fragments, can be accomplished using routine techniques known in the art. For instance, digestion can be performed using papain. Examples of papain digestion are described in WO 94/29348 published December 22, 1994 and U.S. Pat. No.4,342,566. Papain digestion of antibodies typically produces two identical antigen binding fragments, called Fab fragments, each with a single antigen binding site, and a residual Fc fragment. Pepsin treatment yields a fragment that has two antigen combining sites and is still capable of cross-linking antigen.

A further aspect of the invention provides a nucleic acid (for example a polynucleotide) encoding for a peptide of the invention. The polynucleotide may be, for example, DNA, cDNA, PNA, RNA or combinations thereof, either single- and/or double-stranded, or native or stabilized forms of polynucleotides, such as, for example, polynucleotides with a phosphorothioate backbone and it may or may not contain introns so long as it codes for the polypeptide. Of course, only polypeptide that contain naturally occurring amino acid residues joined by naturally occurring peptide bonds are encodable by a polynucleotide. A still further aspect of the invention provides an expression vector capable of expressing a polypeptide according to the invention.

The term "nucleotide sequence" refers to a heteropolymer of deoxyribonucleotides. The nucleotide sequence coding for a particular peptide, oligopeptide, or polypeptide may be naturally occurring or they may be synthetically constructed. Generally, DNA segments encoding the peptides, polypeptides, and proteins of this invention are assembled from cDNA fragments and short oligonucleotide linkers, or from a series of oligonucleotides, to provide a synthetic gene that is capable of being expressed in a recombinant transcriptional unit comprising regulatory elements derived from a microbial or viral operon.

A desirable method of modifying the DNA encoding the polypeptide of the invention uses the polymerase chain reaction as disclosed by Saiki et al., (Primer-directed enzymatic amplification of DNA with a thermostable DNA polymerase. Science. 1988 Jan 29;239(4839):487-91). This method may be used for introducing the DNA into a suitable vector, for example by engineering in suitable restriction sites, or it may be used to modify the DNA in other useful ways as is known in the art. If viral vectors are used, pox- or adenovirus vectors are preferred.

The DNA (or in the case of retroviral vectors, RNA) may then be expressed in a suitable host to produce a polypeptide of the invention. Thus, the DNA encoding the polypeptide of the invention may be used in accordance with known techniques, appropriately modified in view of the teachings contained herein, to construct an expression vector, which is then used to transform an appropriate host cell for the expression and production of the polypeptide of the invention. Such techniques include those disclosed in US Patent Nos. 4,440,859, 4,530,901, 4,582,800, 4,677,063, 4,678,751, 4,704,362, 4,710,463, 4,757,006, 4,766,075, and 4,810,648.

The DNA (or in the case of retroviral vectors, RNA) encoding the polypeptide of the invention may be joined to a wide variety of other DNA sequences for introduction into an appropriate host. The companion DNA will depend upon the nature of the host, the manner of the introduction of the DNA into the host, and whether episomal maintenance or integration is desired.

Generally, the DNA is inserted into an expression vector, such as a plasmid, in proper orientation and correct reading frame for expression. If necessary, the DNA may be linked to the appropriate transcriptional and translational regulatory control nucleotide sequences recognized by the desired host, although such controls are generally available in the expression vector. The vector is then introduced into the host through standard techniques. Generally, not all of the hosts will be transformed by the vector. Therefore, it will be necessary to select for transformed host cells. One selection technique involves incorporating into the expression vector a DNA sequence with any necessary control elements, that codes for a selectable trait in the transformed cell, such as antibiotic resistance.

Alternatively, the gene for such selectable trait can be on another vector, which is used to co-transform the desired host cell. Host cells that have been transformed by the recombinant DNA of the invention are then cultured for a sufficient time and under appropriate conditions known to those skilled in the art in view of the teachings disclosed herein to permit the expression of the polypeptide, which can then be recovered.

Many expression systems are known, including bacteria (for example *E. coli* and *Bacillus subtilis*), yeasts (for example *Saccharomyces cerevisiae*), filamentous fungi (for example *Aspergillus spec*.), plant cells, animal cells and insect cells. Preferably, the system can be mammalian cells such as CHO cells available from the ATCC Cell Biology Collection.

A typical mammalian cell vector plasmid for constitutive expression comprises the CMV or SV40 promoter with a suitable poly A tail and a resistance marker, such as neomycin. One example is pSVL available from Pharmacia, Piscataway, NJ, USA. An example of an inducible mammalian expression vector is pMSG, also available from Pharmacia. Useful yeast plasmid vectors are pRS403-406 and pRS413-416 and are generally available from Stratagene Cloning Systems, La Jolla, CA 92037, USA. Plasmids pRS403, pRS404, pRS405 and pRS406 are Yeast Integrating plasmids (YIps) and incorporate the yeast selectable markers HIS3, TRP1, LEU2 and URA3. Plasmids pRS413-416 are Yeast Centromere plasmids (Ycps). CMV promoter-based vectors (for example from Sigma-Aldrich) provide transient or stable expression, cytoplasmic expression or secretion, and N-terminal or C-terminal tagging in various combinations of FLAG, 3xFLAG, c*-myc* or MAT. These fusion proteins allow for detection, purification and analysis of recombinant protein. Dual-tagged fusions provide flexibility in detection.

The present invention also relates to a host cell transformed with a polynucleotide vector construct of the present invention. The host cell can be either prokaryotic or eukaryotic. Bacterial cells may be preferred prokaryotic host cells in some circumstances and typically are a strain of *E*. *coli* such as, for example, the *E. coli* strains DH5 available from Bethesda Research Laboratories Inc., Bethesda, MD, USA, and RR1 available from the American Type Culture Collection (ATCC) of Rockville, MD, USA (No ATCC 31343). Preferred eukaryotic host cells include yeast, insect and mammalian cells, preferably vertebrate cells such as those from a mouse, rat, monkey or human fibroblastic and colon cell lines. Yeast host cells include YPH499, YPH500 and YPH501, which are generally available from Stratagene Cloning Systems, La Jolla, CA 92037, USA. Preferred mammalian host cells include Chinese hamster ovary (CHO) cells available from the ATCC as CCL61, NIH Swiss mouse embryo cells NIH/3T3 available from the ATCC as CRL 1658, monkey kidney-derived COS-1 cells available from the ATCC as CRL 1650 and 293 cells which are human embryonic kidney cells. Preferred insect cells are Sf9 cells which can be transfected with baculovirus expression vectors. An overview regarding the choice of suitable host cells for expression can be found in, for example, the textbook of Paulina Balbás and Argelia Lorence "Methods in Molecular Biology Recombinant Gene Expression, Reviews and Protocols," Part One, Second Edition, ISBN 978-1-58829-262-9, and other literature known to the person of skill.

Transformation of appropriate cell hosts with a DNA construct of the present invention is accomplished by well known methods that typically depend on the type of vector used. With regard to transformation of prokaryotic host cells, see, for example, Cohen et al (1972) Proc. Natl. Acad. Sci. USA 69, 2110, and Sambrook et al (1989) Molecular Cloning, A Laboratory Manual, Cold Spring Harbor Laboratory, Cold Spring Harbor, NY. Transformation of yeast cells is described in Sherman et al (1986) Methods In Yeast Genetics, A Laboratory Manual, Cold Spring Harbor, NY. The method of Beggs (1978) Nature 275,104-109 is also useful. With regard to vertebrate cells, reagents useful in transfecting such cells, for example calcium phosphate and DEAE-dextran or liposome formulations, are available from Stratagene Cloning Systems, or Life Technologies Inc., Gaithersburg, MD 20877, USA. Electroporation is also useful for transforming and/or transfecting cells and is well known in the art for transforming yeast cell, bacterial cells, insect cells and vertebrate cells.

Successfully transformed cells, i.e. cells that contain a DNA construct of the present invention, can be identified by well-known techniques such as PCR. Alternatively, the presence of the protein in the supernatant can be detected using antibodies as described herein.

It will be appreciated that certain host cells of the invention are useful in the preparation of the peptides of the invention, for example bacterial, yeast and insect cells. However, other host cells may be useful in certain therapeutic methods. For example, antigen-presenting cells, such as dendritic cells, may successfully be used to express the peptides of the invention such that they may be loaded onto appropriate MHC molecules. Thus, the current invention provides a host cell comprising a nucleic acid or an expression vector according to the invention. In a preferred embodiment, the host cell is an antigen-presenting cell, in particular a dendritic cell or an equivalent antigen-presenting cell.

Another aspect of the present invention then relates to isolated T cells comprising T-cell receptors (TCR), which specifically bind to a polypeptide according to the invention.

Disclosed is an *in vitro* method for producing activated cytotoxic T lymphocytes (CTL). The method comprising contacting *in vitro* CTL with antigen-loaded human class I MHC molecules expressed on the surface of a suitable antigen-presenting cell for a period of time sufficient to activate the CTL in an antigen-specific manner. The antigen is the mHAg peptide according to the invention. Preferably, a sufficient amount of the antigen is used with an antigen-presenting cell.

In yet another aspect the objective of the invention is further solved by an *in vitro* method for generating T cells, comprising the steps of a) providing a first cell that expresses a peptide according to the present invention, preferably wherein the peptide is full-length CYBA-72Y/B15, b) bringing a population of peripheral blood mononuclear cells (PBMCs) into contact with said first cell, and thereby stimulating said PBMCs, and c) selecting out of the population of stimulated PBMCs T cells which have the ability to recognize a cell expressing the peptide used in (a). In this aspect, the MHC is preferably MHC class I.

In one preferred embodiment of the method described above, said antigen-presenting cell and said PBMCs are autologous cells derived from one cancer patient. PBMC-derived T cells are usable for re-injection into the patient as a treatment-agent against the cancer the patient is suffering from.

In case of a MHC class I epitope used as an antigen, the CTL are CD8-positive T cells. The MHC class I molecules may be expressed on the surface of any suitable cell, and it is preferred that this cell does not naturally express MHC class I molecules (in which case the cell is transfected to express such a molecule). Allogeneic cells may also be used in the preparation of CTL and an exemplary method is described in detail in WO 97/26328. For example, in addition to Drosophila cells and T2 cells, other cells may be used to present antigens such as CHO cells, baculovirus-infected insect cells, bacteria, yeast, vaccinia-infected target cells.

If an antigen-presenting cell is transfected to express such an epitope preferably the cell comprises an expression vector capable of expressing a peptide containing SEQ ID NO 1.

The activated CTL that are directed against the peptide of the invention are useful in therapy. Thus, a further aspect of the invention provides activated CTL obtainable by the foregoing methods of the invention. Activated CTLs, produced by the above method will selectively recognize a cell that aberrantly expresses a polypeptide comprising an amino acid sequence of SEQ ID NO 1.

In a further aspect, the invention provides a T cell comprising a Tcell receptor (TCR) or a fragment thereof, which binds to a peptide according to the invention. A TCR is a heterodimeric cell surface protein of the immunoglobulin superfamily, which is associated with invariant proteins of the CD3 complex involved in mediating signal transduction. TCRs exist in αβ and γδ forms, which are structurally similar but have quite distinct anatomical locations and probably functions. The extracellular portion of native heterodimeric αβTCR consists of two polypeptides, each of which has a membrane-proximal constant domain, and a membrane-distal variable domain. Each of the constant and variable domains includes an intra-chain disulfide bond. The variable domains contain the highly polymorphic loops analogous to the complementarity determining regions (CDRs) of antibodies.

The T cells of the present invention may be used as active ingredients of a therapeutic composition. Thus, the invention also provides a method of killing target cells in a patient whose target cells aberrantly express a polypeptide comprising an amino acid sequence of the invention, the method comprising administering to the patient an effective number of T cells as defined above.

Yet another aspect of the present invention then relates to a pharmaceutical composition, comprising a peptide according to the present invention, or a nucleic acid according to the present invention, a vector according to the present invention, a cell according to the present invention, or an isolated T cell according to the present invention, preferably in the form of a vaccine.

The pharmaceutical composition comprises the polypeptide either in the free form or in the form of a pharmaceutically acceptable salt.

As used herein, "a pharmaceutically acceptable salt" refers to a derivative of the disclosed peptides wherein the peptide is modified by making acid or base salts of the agent. For example, acid salts are prepared from the free base (typically wherein the neutral form of the drug has a neutral -NH₂ group) involving reaction with a suitable acid. Suitable acids for preparing acid salts include both organic acids, e.g., acetic acid, propionic acid, glycolic acid, pyruvic acid, oxalic acid, malic acid, malonic acid, succinic acid, maleic acid, fumaric acid, tartaric acid, citric acid, benzoic acid, cinnamic acid, mandelic acid, methanesulfonic acid, ethanesulfonic acid, p-toluenesulfonic acid, salicylic acid, and the like, as well as inorganic acids, e.g., hydrochloric acid, hydrobromic acid, sulfuric acid, nitric acid phosphoric acid and the like. Conversely, preparation of basic salts of acid moieties which may be present on a peptide are prepared using a pharmaceutically acceptable base such as sodium hydroxide, potassium hydroxide, ammonium hydroxide, calcium hydroxide, trimethylamine or the like.

In an especially preferred embodiment the pharmaceutical compositions comprise the peptide as salts of acetic acid (acetates) or hydrochloric acid (chlorides).

One particularly preferred embodiment of the present invention relates to the peptide according to the description herein before, for use in medicine, specifically for use in a method of treatment of the human or animal body by surgery or therapy, or diagnostic methods applied to the human or animal body.

Further provided according to the present invention is the peptide as described herein, for use in the prevention and/or treatment of a disease selected from transplant rejection, such as, for example, graft versus host disease, malignancies, such as, for example, leukemias, such as, for example, acute lymphoblastic leukemia (ALL), acute myeloid leukemia (AML), chronic lymphocytic leukemia (CLL), chronic myelogenous leukemia (CML), accelerated phase or blast crisis; lymphomas, such as, for example, Hodgkin's disease, non-Hodgkin's lymphoma, and myelomas, such as, for example, multiple myeloma (Kahler's disease), and relapses thereof. Preferred is the use, wherein the cells of said malignancy express or aberrantly express the protein CYBA-72Y.

To date, a relapse is the most common reason for a failure of therapy and mortality following an HSCT. The risk for a relapse is dependent on the underlying disease, the state of the disease before allo-HSCT, and the kind of GvHD prophylaxis. Patients that are transplanted in the first remission of an acute leukemia have a relapse risk of 20 - 30%.

Thus, further preferred is the use, wherein said malignancy does not respond to prolonged treatment with chemotherapy, and/or is resistant to chemotherapy.

Disclosed is also a method for preventing or treating a disease selected from transplant rejection, such as, for example, graft-versus-host disease, transplant rejections in the context of an allo-HSCT, malignancies, such as, for example, leukemias, such as, for example, acute lymphoblastic leukemia (ALL), acute myeloid leukemia (AML), chronic lymphocytic leukemia (CLL), chronic myelogenous leukemia (CML), accelerated phase or blast crisis; lymphomas, such as, for example, Hodgkin's disease, non-Hodgkin's lymphoma, and myelomas, such as, for example, multiple myeloma (Kahler's disease), and relapses thereof, comprising administering to a subject in need thereof a polypeptide according to the present invention, or a nucleic acid according to the present invention, a vector according to the present invention, a cell according to the present invention, a monoclonal antibody or an isolated T cell according to the present invention, preferably in the form of the pharmaceutical composition according to the present invention, more preferably in the form of a vaccine according to the present invention.

A therapeutically effective amount will be an amount sufficient to induce an immune response, in particular an activation of a subpopulation of CTLs. A person skilled in the art may easily determine whether an amount is effective by using standard immunological methods, such as those provided in the examples of the present specifications. Another way of monitoring the effect of a certain amount of the pharmaceutical composition is to observe the growth of the tumor treated and/or its recurrence.

In a particularly preferred embodiment of the present invention the pharmaceutical composition is used as an anti-cancer vaccine. The vaccine according to the present invention can be given *inter alia* intravenously, intraperitoneally, intranasally, intradermally, subcutaneously or intramuscularly.

A preventive treatment in the context of the herein described invention is of benefit possibly mainly to persons who have an increased risk of developing a malignancy, because, for example, they are hereditarily predisposed or because they have already had a malignancy before. In another embodiment, a preventive treatment is of benefit for a patient suffering from a disease with increased risk of having developed or developing resistance to immune rejection, by e.g. immune escape via the alteration of the function and/or expression of HLA class I/II complexes.

In another aspect the present disclosure describes a method for inducing tolerance for transplants in a subject that is in need of tolerance for transplants, comprising administering to said subject a tolerance-inducing amount of a peptide consisting of amino acid sequence according to SEQ ID NO: 1, whereby rejection of transplants or GvHD is reduced. The subject preferably is a mammal, such as a human patient.

The CTLs of the invention may be used as active ingredients in a therapeutic composition. Thus the invention also provides a method of killing target cells in a patient where the target cells aberrantly express a polypeptide comprising an amino acid sequence of the invention. The method comprises administering to the patient an effective number of CTLs as defined above. The CTLs administered to the patient may be derived from the patient and activated as described above (i.e. they are autologous CTLs). Alternatively, the CTLs are not from the patient but are from an HLA-matched individual. Of course, preferably the donor is a healthy individual. By "healthy individual" it is meant that the individual is generally in good health, preferably has a competent immune system and, more preferably, is not suffering from any disease that can be readily tested for, and detected.

In addition to being useful for treating cancer, the polypeptide of the present invention is also useful as a diagnostic agent, e.g for selection of donor/recipient pairs in allo-HSCT.

The detection of claimed peptides or encoding nucleic acids on diseased tissue specimen can enable the decision about the benefit of therapies involving the immune system, such as allo-HSCT, especially if T lymphocytes are known or expected to be involved in the mechanism of action.

Claimed peptides or encoding nucleic acids might be used to analyze lymphocyte responses such as T cell responses or antibody responses against the peptide or the peptide complexed to MHC molecules. These lymphocyte responses can be used as prognostic markers for the decision on further therapy steps. These responses can also be used as surrogate markers in immunotherapy approaches aiming to induce lymphocyte responses by different means, e.g. vaccination of protein, nucleic acids, autologous cellular materials, adoptive transfer of lymphocytes. In gene therapy settings involving the transfer of the CYBA-72Y/B15-reactive TCR, lymphocyte responses against peptides can be considered in the assessment of side effects. Monitoring of lymphocyte responses is also a valuable tool for follow-up examinations of transplantation therapies, e.g. for the detection of GvHD.

Further provided according to the present invention is the use of a polypeptide as described above, a nucleic acid as described above, a vector as described above, an antibody as described above, a T cell or antigen-presenting cell as described above, in the preparation of a medicament for treating a disease selected from transplant rejection, such as, for example, GvHD, malignancies, such as, for example, leukemias, such as, for example, acute lymphoblastic leukemia (ALL), acute myeloid leukemia (AML), chronic lymphocytic leukemia (CLL), chronic myelogenous leukemia (CML), accelerated phase or blast crisis; lymphomas, such as, for example, Hodgkin's disease, non-Hodgkin's lymphoma, and myelomas, such as, for example, multiple myeloma (Kahler's disease), and relapses thereof, or in the preparation of a diagnostic for diagnosing malignancies as above. The malignancy may be a mammalian cancer. In particular, the malignancy may be a human cancer. The medicament may be an anti-cancer vaccine as described herein.

In another aspect the invention further provides an *in vitro* method for diagnosing the suitability of a donor and/or recipient for an allo-HSCT, comprising the step of determining the presence or absence of the mHAg according to SEQ ID No. 1 in a biological sample from a donor and/or recipient of an allo-HCST. Respective methods are known to the person of skill, and preferably comprise a detection based on antibodies, PCR and/or RT-PCR as described herein.

The mHAg CYBA-72Y/B15 and additional epitopes from CYBA can be used in transplant medicine and diagnostics as well as in the immunotherapy of malignancies. In order to increase a CYBA-72Y-specific immune response, patients (recipients) and donors can be vaccinated against immunogenic CYBA-72Y/B15, wherein the antigen can be administered in the form of *in vitro* transcribed (IVT) mRNA, a recombinant protein, synthetic peptides or in recombinant viral vector. For the adaptive transfer of CYBA-72Y-specific T cells, lymphocytes can be expanded through stimulation with the peptide as identified or using APCs that were transfected with CYBA-encoding nucleic acids. The advantages of such a transfection of the APCs are the natural processing and surface presentation of multiple peptides, leading to a broader repertoire of reactive T cells restricted by various HLA alleles. The T-cell receptors as isolated from CYBA-72Y/B15-reactive T-lymphocytes can be transferred retrovirally or in form of an IVT-RNA both into donor as well as into patient cells, in order to improve the GvL/GvT effect.

In order to monitor the response to the immunotherapy, antigen-specific T-cells can be detected in the blood of patients by using HLA/peptide-multimers or cytokine-based reaction assays (e.g. interferon-gamma-ELISpot assay), whereby antigen-presenting cells are transfected with the antigen or loaded with peptides.

In case of a CYBA-72Y-mediated GvHD, peptide-analogs can be used therapeutically that specifically block or inhibit T cells. For GvHD prophylaxis, before an allo-HSCT or a DLI a depletion of specific T cells can be performed *in vitro.* This can be achieved using peptides or multimers, or through the stimulation of the lymphocytes with peptide-loaded or antigen-transfected APCs. The stimulated lymphocytes can then be removed from the transplant or the DLI preparation using antibodies binding to membrane-located activation markers or with HLA/peptide multimers.

The present invention will now be further described in the following examples with reference to the accompanying figures and sequences, nevertheless, without being limited thereto. For the purposes of the present invention. The Figures and Sequences show:

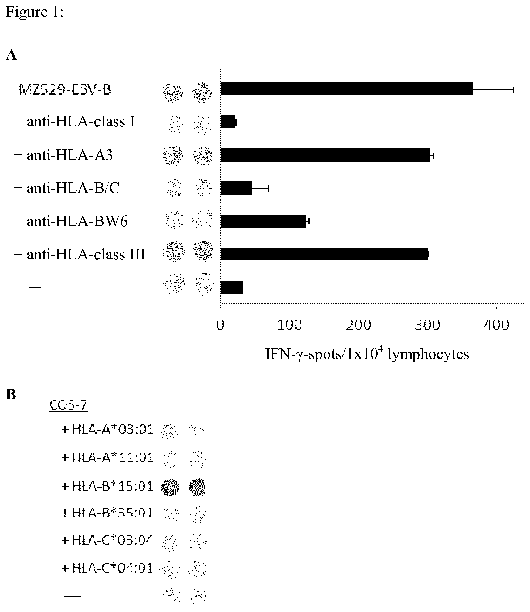

Figure 1: HLA restriction of miniMLLC 2E8. A) MZ529-EBV-B-cells (5×10⁴ cells/well) were co-incubated in the presence of HLA-specific antibodies at concentrations mediating complete blocking of control-T lymphocytes with corresponding HLA restriction, with the miniMLLC 2E8 (1×10⁴ cells/TU) in duplicate reactions in a 20h-IFN-gamma ELISpot assay. B) COS-7-cells (2×10⁴ cells/well) were transfected with the HLA alleles of patient MZ529-AML and Buffy Coat donor 730 and tested in duplicate reactions for recognition by 2E8 (2x10⁴ cells/well) in a 20h-IFN-gamma ELISpot assay.

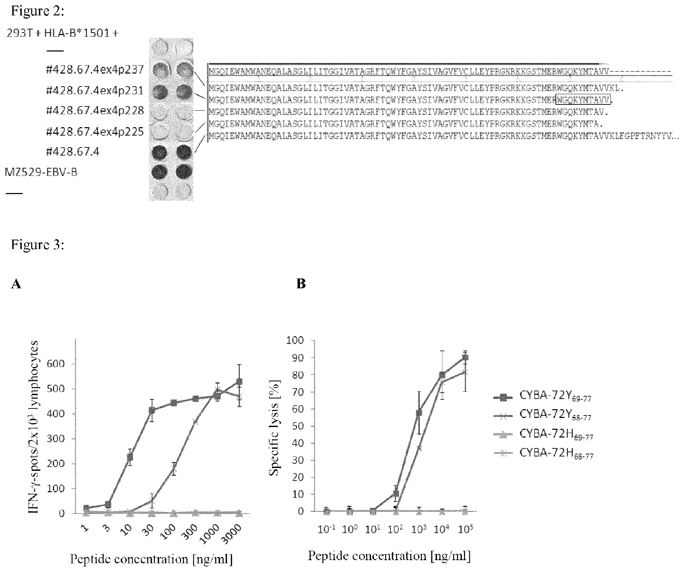

Figure 2: Fragment analysis of the cDNA clone #428.67.4 for the identification of the peptide-coding region. 3'-fragments with a length difference of one codon (i.e. one amino acid) were generated from the cDNA clone #428.67.4 encoding the full-length CYBA-72Y, and were tested after co-transfection with HLA-B*15:01 into 293T cells (2x10⁴ cells/well) in a 20h-IFN-gamma ELISpot assay for recognition by 2E8 (1.5x10⁴ cells/well). MZ529-EBV-B cells (5x10⁴ cells/well) served as positive control target.
Figure 3: Peptide recognition by the miniMLLC 2E8. (A) For a 20h-IFN-gamma ELISpot assay, 293T cells (2x10⁴ cells/well) were transfected with HLA-B*15:01, were loaded with the indicated synthetic CYBA peptides at different concentrations, and were tested in duplicate reactions for recognition by 2E8 (2x10⁴ cells/well). (B) For a 48h-⁵¹Cr-release assay, HLA-B*15:01-expressing L721.221-cells (2x10⁴ cells/well) were loaded with the indicated synthetic CYBA peptides at different concentrations, and co-incubated in duplicate experiments with 2E8 (2x10⁴ cells/well).

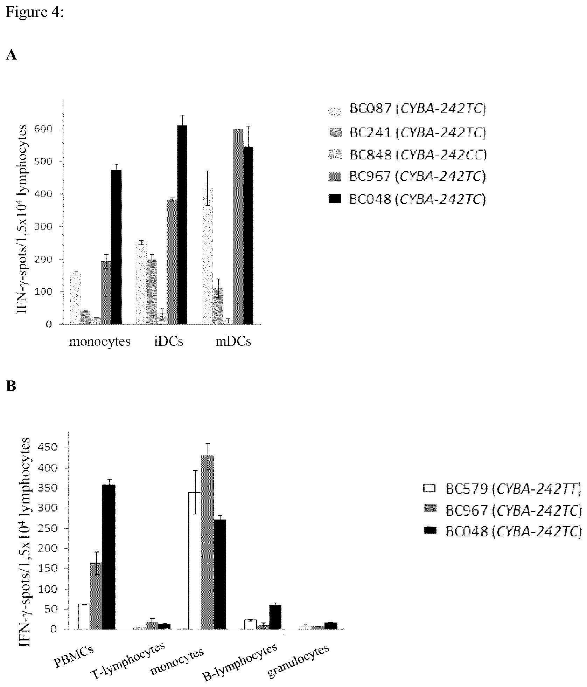

Figure 4: Recognition of non-malignant hematopoietic cells from HLA-B*15:01-positive BC donors by the CYBA-72Y/B15-specific miniMLLC 2E8. (A) From PBMCs of HLA-B*15:01-positive BC donors, who -with the exception of BC848 - carried at least one *CYBA-242T* allele (coding for CYBA-72Y), monocytes were purified using plastic adherence and differentiated to mature dendritic cells (mDCs) using cytokines. In duplicates 5x10⁴ (BC087, BC848, BC967) or 3x10⁴ (BC241, BC048) cells per well were tested for recognition by 2E8 (1.5x10⁴ cells/well) in 20h-IFN-gamma ELISpot assays. (B) From PBMCs of HLA-B*15:01-positive BC-donors with at least one *CYBA-242T* allele (encoding CYBA-72Y) monocytes (CD14⁺), B lymphocytes (CD19⁺, CD20⁺) and T lymphocytes (CD4⁺, CD8⁺) were isolated using magnetic microbeads coated on their surfaces with the respective anti-CD-antibodies. Granulocytes were isolated from whole blood by means of anti-CD66abce-beads (BC048) or density gradient centrifugation (BC579, BC967). In duplicates 3x10⁴ PBMCs, T cells, B cells and granulocytes or 0.7x10⁴ monocytes per well of BC048 as well as 1x10⁴ cells/well of BC579 and BC967 were co-incubated with 2E8 (1.5 x10⁴ cells/well) in 20h-IFN-gamma ELISpot assays.

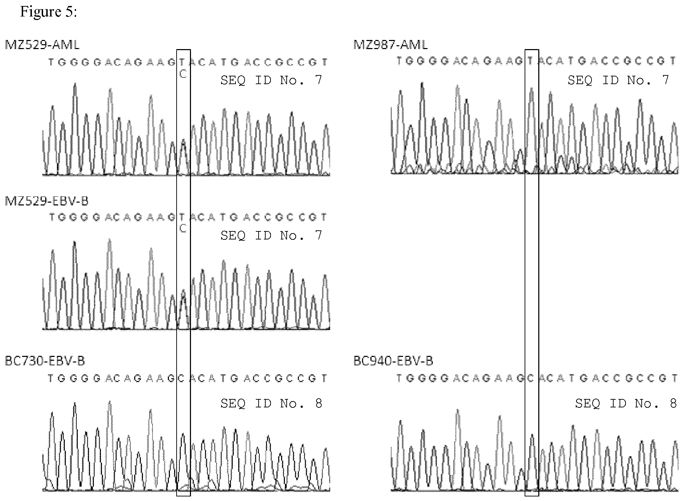

Figure 5: *CYBA-242* genotyping of patients MZ529 and MZ987 and of buffy coat (BC) donors 730 and 940. RNA that was isolated from the cells of AML-patients MZ529 and MZ987 and BC donors 730 and 940 was reverse-transcribed into cDNA. A CYBA-cDNA-fragment containing the polymorphism rs4673 at nucleic acid position 242 was amplified and sequenced.

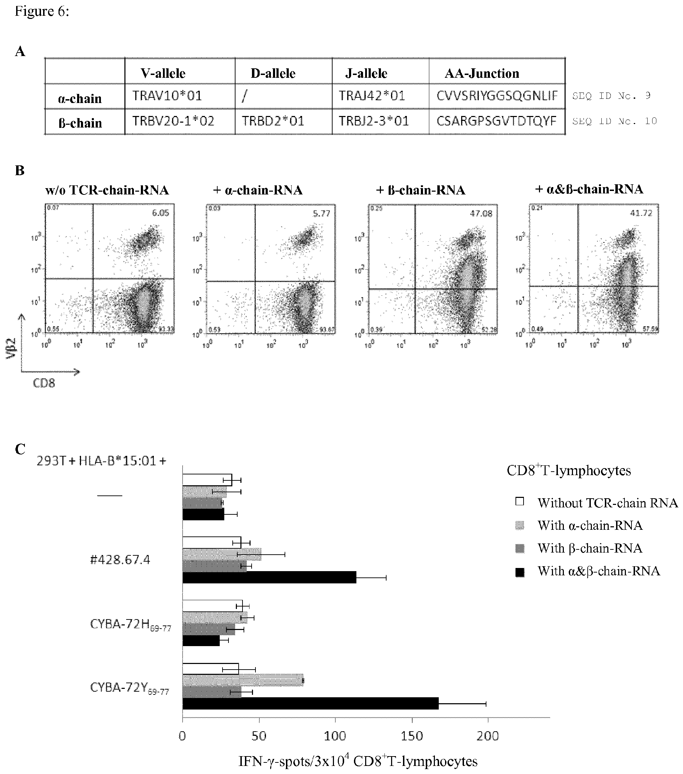

Figure 6: Functionality of the T-cell receptor cloned from miniMLLC 2D10 (from BC940). *In vitro* transcribed mRNA for both the alpha- and the beta-chain of the T-cell receptor (TCR) of miniMLLC 2D10 was electroporated into CD8⁺ lymphocytes of BC donor 560 (not carrying a *CYBA-242T* allele). A) Typing of the alpha- and the beta-chain of the T-cell receptor (TCR) of miniMLLC 2D10. B) Flow cytometry was used in order to show the expression of the beta-chain with an antibody against Vβ2 on the CD8⁺ recipient cells. C) 293T cells transfected with HLA-B*15:01 (2x10⁴ cells/well) were co-transfected with cDNA-clone #428.67.4, or loaded with CYBA peptides CYBA-72Y₆₉₋₇₇ and CYBA-72H₆₉₋₇₇ at a final concentration of 5 ug/ml, and tested in a 20h-IFN-gamma ELISpot assay for recognition by CD8⁺ donor lymphocytes (3x10⁴ cells/well) electrophorated with both (alpha and beta) or with single (alpha or beta) chains of the 2D10-T-cell receptor.
SEQ ID NO. 1 shows the amino acid sequence of the peptide according to the present invention: GQK**Y**MTAVV (mutated amino acid in bold).
SEQ ID NO. 2 shows the amino acid sequence of CYBA with the peptide of the invention underlined, and the mutated amino acid in bold.

### EXAMPLES

### Identification of the mHAg CYBA-72Y/B15

In the model of AML patient MZ529, naive CD45RA⁺CD8⁺ T lymphocytes derived from HLA-class I-identical healthy buffy coat donor BC730 were stimulated using MZ529 leukemic cells. The thus produced population of AML-reactive T cells, generated as so-called mini-MLLCs (mini-mixed lymphocyte-leukemia culture; Albrecht *et al.,* 2011) were restricted by HLA-B*15:01. The yet unknown target antigen CYBA-72Y/B15 was identified via cDNA expression cloning using these T cells. Because of the single nucleotide polymorphism (SNP) rs4673 of the gene *CYBA* (two alleles of CYBA, *CYBA-242T* and *CYBA-242C* exist that encode tyrosine (Y) or histidine (H) at amino acid position 72, respectively. An allogeneic T cell response against CYBA-72Y/B15 was observed in an additional independent AML model, where T lymphocytes derived from the HLA-class I-identical donor BC940 were stimulated with the leukemic cells of AML patient MZ987. CYBA-72Y/B15-specific T cell responses were found in three of five tested HLA-B*15:01-positive BC-donors that were homozygous for CYBA-72H (H/H). The peptide that was recognized by all T lymphocytes at the lowest concentration had the sequence GQKYMTAVV, and thus comprised amino acids 69-77 (see SEQ ID No: 2), whereas the homologous peptide from CYBA-72H did not lead to any reactivity, even at high concentrations. T lymphocytes against CYBA-72Y/B 15 recognized leukemic cells from eight of twelve HLA-B*15:01-positive AML patients (FAB-subtypes: M1, M2, M4, M5). Since the gene *CYBA* encodes for a component of the microbicidal oxidase system of phagocytotic cells, it is likely that it is primarily expressed in cells of the hematopoietic system. Subtypes of leukocytes that were purified from HLA-B*15:01-positive BC donors carrying the *CYBA-242T* allele strongly recognized monocytes and the respective dendritic cells, whereas non-transformed B cells were recognized to a much lower extent, and granulocytes and T-lymphocytes were not recognized. The allele *CYBA-242T* encoding CYBA-72Y was detected in 56% of all tested healthy donors and tumor patients (n=408). When also taking into account the frequency of the presenting allele *HLA-B*15:01,* about 4.5% of all Caucasians can be calculated to carry the newly identified immunogenic mHAg/HLA combination. In the context of an allo-HCST an immunogenic CYBA-72Y mismatch does not necessarily lead to severe GvHD. The mHAg CYBA-72Y/B15 as described herein thus seems suitable to mediate the preferential elimination of the recipients' hematopoiesis including myeloid leukemia cells in the context of an allo-HSCT.

Via *in vitro* stimulation of naive CD45RA⁺CD8⁺ T lymphocytes derived from healthy buffy coat donor BC730 with HLA-class I-identical AML cells of patient MZ529, AML-reactive miniMLLCs were generated that also recognized EBV-transformed B cells of the patient (Albrecht et al., 2011). Using blocking tests with anti-HLA antibodies and the co-transfection of all HLA-class I-alleles of the patient MZ529 together with *CYBA-242T* into COS-7-cells, HLA-B*15:01 was identified as the restriction molecule of the four miniMLLCs 2E8, 4D3, 7H9, and 7G10 (Figure 1 for 2E8). Surprisingly, even transfection of only HLA-B*15:01 led to recognition of COS-7-cells. 293T-cells were not recognized after transfection of HLA-B*15:01 only and were therefore used as recipient cell line in cDNA expression screening experiments (see below).

A cDNA expression library was constructed in vector pcDNA3.1/RfA/Dest. from the mRNA of EBV-B-cells from patient MZ529. The library was first divided into pools of 100 cDNA clones per transfection unit (100x pools). In a first screening, the 100x pools were co-transfected with HLA-B*15:01-cDNA into 293T-cells, and screened for recognition by miniMLLCs. Pool #428 was among the recognized pools. This pool was then further subcloned into 8x pools, each consisting of 8 cDNA clones per transfection unit, which were then co-transfected with HLA-B*15:01 into 293T cells, and again tested for T-cell recognition in analogy to the 100x pools. 8x pool #428.67 was positive.

Cloning of the 8x pool #428.67 and the testing of the resulting cDNA clones finally led to identification of the cDNA clone #428.67.4. The sequencing of this cDNA clone showed that it contained the full length cDNA encoding cytochrome b-245 alpha-polypeptide (CYBA, p22phox). Database analyses revealed that the cDNA as recognized by 2E8 represented an allele of *CYBA* that, due to a known single nucleotide polymorphism (rs4673: *CYBA-242T*/*C*), coded for tyrosine (Y) instead of histidine (H) at amino acid position 72. The antigen as recognized by 2E8 was therefore designated CYBA-72Y and is encoded by *CYBA-242T.* In turn, CYBA-72H encoded by *CYBA-242C* was not recognized by 2E8.

Cytochrome b-245 alpha-polypeptide (CYBA, or p22phox) encodes the membrane-bound small subunit of the NADPH-oxidase complexes 1-4 (NOX 1-4) that produce reactive oxygen radicals (ROS). In addition to a function in the removal of pathogens, ROS play a role in signal and differentiation processes as well as in the regulation of gene expression (Bedard and Krause The NOX family of ROS-generating NADPH oxidases: physiology and pathophysiology. Physiol Rev. 2007 Jan;87(1):245-313; and Brar et al. An NAD(P)H oxidase regulates growth and transcription in melanoma cells. Am J Physiol Cell Physiol. 2002 Jun;282(6):C1212-24). In agreement with their broad spectrum of functions, the NADPH oxidases are found in a multitude of cell types (e.g. phagocytes, endothelial and muscle cells, fibroblasts).

Via the stepwise 3'-fragmentation of cDNA clone #428.67.4., valine (V) at amino acid position 77 was identified as C-terminal end of the peptide antigen as recognized by 2E8 (Figure 2). Starting from this, the synthetic CYBA-72Y peptides 68-77 (10mer; WGQKYMTAVV) and 69-77 (9mer; GQKYMTAVV) were tested for recognition by T cells using an IFN-gamma-ELISpot assay, and by means of a ⁵¹Cr-(chromium) release test. Both peptides contained the polymorphic amino acid 72Y. As can be seen in Figure 3, both peptides were recognized, whereby, nevertheless, the 9mer was always recognized at lower concentrations, and thus better, than the 10mer. The homologous peptides from CYBA-72H were not recognized. Also the other HLA-B*15:01- restricted miniMLLCs (4D3, 7G9, 7H1) of donor 730 recognized CYBA-72Y with the same specificity as 2E8.

T-cells against CYBA-72Y/B15 were identified in a different independent model system. Naive CD45RA⁺CD8⁺ T lymphocytes derived from buffy coat donor BC940 were stimulated with AML cells of the HLA-class I-matching AML patient MZ987. The resulting miniMLLCs 2B8 und 2D10 recognized the AML- and EBV-B-cells of patient MZ529 and were restricted by HLA-B*15:01. It was found that they had the same peptide specificity as the T-cells directed against CYBA-72Y of BC-donor 730 (not shown).

Since the expression of CYBA was seen primarily in hematopoietic cells (http://www.proteinatlas.org/search/CYBA), the recognition of non-malignant hematopoietic sub-populations of BC donors that were HLA-B*15:01 positive and carried at least one *CYBA-242T-allele* was analyzed with CYBA-72Y/B15-reactive T lymphocytes. Monocytes, immature and mature dendritic cells were recognized (Figure 4A). The recognition of B cells was weakly positive in two of three donors, while T lymphocytes and granulocytes were not recognized at all (Figure 4B).

In cross-reactivity analyses, CYBA-72Y/B15-reactive T lymphocytes recognized AML cells from eight of twelve AML patients as well as three of five tested melanoma cell lines. The recognition of allogeneic malignant cells depended on the presence of *HLA-B*15:01* and *CYBA-242T.* An increased expression of CYBA has been described also in other malignomas than AML, such as acute lymphatic leukemia, prostate carcinoma, and melanoma (Floriano Sanchez et al., Evaluation of the expression of p22 phox subunit of NADPH oxidase (NOX) in prostate cancer and benign prostatic hyperplasia: a comparative study. Actas Urol Esp. 2010 Apr;34(4):340-5; He et al., Expression of WAVE1 and p22phox in children with acute lymphocytic leukemia and the relationship of WAVE 1 with oxidative stress. Zhongguo Dang Dai Er Ke Za Zhi. 2009 Feb;11 (2):88-92; Fried and Arbiser, 2008; The reactive oxygen-driven tumor: relevance to melanoma. Pigment Cell Melanoma Res. 2008 Apr;21(2):117-22; Brar et al., An NAD(P)H oxidase regulates growth and transcription in melanoma cells. Am J Physiol Cell Physiol. 2002 Jun;282(6):C1212-24; Brar et al., NOX5 NAD(P)H oxidase regulates growth and apoptosis in DU 145 prostate cancer cells. Am J Physiol Cell Physiol. 2003 Aug;285(2):C353-69. Epub 2003 Apr 9), and has been associated with their uncontrolled growth (Weyemi et al., Intracellular expression of reactive oxygen species-generating NADPH oxidase NOX4 in normal and cancer thyroid tissues. Endocr Relat Cancer. 2010 Jan 29;17(1):27-37; Ushio-Fukai and Nakamura, Reactive oxygen species and angiogenesis: NADPH oxidase as target for cancer therapy. Cancer Lett. 2008 Jul 18;266(1):37-52).

Whereas both AML patients MZ529 and MZ987 were heterozygous or homozygous for the CYBA-72Y-encoding allele *CYBA-242T,* respectively, their BC donors (BC730 or BC940) were homozygous for the CYBA-72H-encoding allele *CYBA-242C* (Figure 5). The genotyping as performed using restriction fragment length polymorphism (RFLP)-analysis in 481 healthy subjects and cancer patients showed that 56% of the study group carried at least one allele encoding CYBA-72Y and 6% were homozygous. Similar distribution patterns can be found in the literature (Tahara et al., Genetic variant of the p22PHOX component of NADPH oxidase C242T and the incidence of gastric cancer in Japan. Hepatogastroenterology. 2008 Nov-Dec;55(88):2273-6; Castejon et al., NAD(P)H oxidase p22phox gene C242T polymorphism, nitric oxide production, salt sensitivity and cardiovascular risk factors in Hispanics. J Hum Hypertens. 2006 Oct;20(10):772-9; Mata-Balaquer et al., Angiotensin-converting enzyme and p22(phox) polymorphisms and the risk of coronary heart disease in a low-risk Spanish population. Int J Cardiol. 2004 Jun;95(2-3):145-51; Wolf et al., No association between a genetic variant of the p22(phox) component of NAD(P)H oxidase and the incidence and progression of IgA nephropathy. Nephrol Dial Transplant. 2002 Aug;17(8):1509-12).

The antigen-presenting HLA allele *HLA-B*15:01* can be found in about 8% of Caucasians. Starting from the group of 481 individuals as analyzed by means of RFLP analysis (see above) it can be calculated that about 4.6% of the German population carry both *HLA-B*15:01* and at least one *CYBA-242T-allele,* and thus express the antigen CYBA-72Y/B15. Nevertheless, allele frequencies vary depending on the ethnic origin. For example, in some Asian populations HLA-B*15:01 was found more frequently as compared to Caucasians.

Nevertheless, in particular in Asian populations the *CYBA-242T* allele is rarer than in Caucasians. Only 13.0 to 20.4% of the Japanese carry *CYBA-242T*m (Kuroda el al., NAD(P)H oxidase p22phox C242T polymorphism and ischemic stroke in Japan: the Fukuoka Stroke Registry and the Hisayama study. Eur J Neurol. 2007 Oct; 4(10):1091-7; Shimo-Nakanishi et al., Functional effects of NAD(P)H oxidase p22(phox) C242T mutation in human leukocytes and association with thrombotic cerebral infarction. Atherosclerosis. 2004 Jul;175(1):109-15). But in the Japanese and in particular in the Indian population the fraction of the *HLA-B*I5:01-* AND *CYBA-242T-*positive individuals is markedly increased with frequencies of 7.8% and 11.6%, respectively. While in the Japanese population this is due to the very high frequency of HLA-B*15:01, it is caused in the Indian population primarily by the very common allele *CYBA-242T* occurring at a frequency of up to 70% (Vibhuti et al., CYP1A1, CYP1A2 and CYBA gene polymorphisms associated with oxidative stress in COPD. Clin Chim Acta. 2010 Apr 2;411(7-8):474-80; Choi et al., Genetic polymorphisms in molecules of innate immunity and susceptibility to infection with Wuchereria bancrofti in South India. Genes Immun. 2001 Aug;2(5):248-53.).

The T cells against CYBA-72Y/B15 as generated in the context of the present invention also recognized CYBA in association with HLA-B*15:07. The antigen was not recognized in association with HLA-B*15:03, -B*15:04, and -B*15:17. Nevertheless, HLA-B*15:07 is much rarer than HLA-B*15:01. In about one quarter (n=6) of allo-HSCTs in HLA-B*15:01-positive donor/recipient-pairs an immunogenic *CYBA*-*242*-mismatch was found, i.e. the patients carried at least one *CYBA-242T* allele, and their donors were homozygous for the *CYBA-242C* allele. Thus, CYBA-72Y-specific T-lymphocytes derived from the donor hematopoiesis should have been able to recognize the antigen on cells of the patient (=recipient). Due to the *in vitro* recognition of AML cells, as found in the context of the present invention, and of certain non-malignant hematopoietic cells by CYBA-72Y/B15-specific T cells a triggering of a GvL effect and the potential elimination of at least a part of the recipient hematopoietic system by CYBA-72Y-reactive donor lymphocytes could be assumed. Regarding normal tissues, in the six transplantation patients identified with an immunogenic *CYBA-242*-mismatch, only three developed a chronic GvHD, and only one of developed acute GvHD. In all cases, the GvHD symptoms could be controlled, and the patients achieved a continuous complete remission.

The TCR chains of the mini-MLLCs directed against CYBA-72Y/B15 from both model systems were analyzed. It was found that the T-cell reactions against the mHAg were at least oligoclonal. In order to prepare for adoptive transfer experiments, the TCR chains were cloned from mini-MLLC 2D10 using TCRalpha- and TCRbeta-specific PCR (Birkholz et al., A fast and robust method to clone and functionally validate T-cell receptors. J Immunol Methods. 2009 Jul 31;346(1-2):45-54). The sequence analysis showed that the alpha-chain contains a TRAV10*01- and a TRAJ42*01-region. The beta-chain contains TRBV20-1*02, TRBD2*01, and TRBJ2-3*01 (Figure 6A). The successful transfer of the specificity and the functionality of the TCRalpha- and TCRbeta-chains cloned from 2D10 was shown by means of RNA transfer into CD8+ cells from healthy BC donors (Figure 6B, C).

## Claims

1. A peptide consisting of the amino acid sequence according to SEQ ID No. 1 (GQK**Y**MTAVV), wherein said peptide is capable of inducing a T-cell response.

2. The peptide according to claim 1, wherein said peptide is capable of binding to an MHC class I molecule.

3. An isolated nucleic acid molecule, encoding for a peptide according to any one of claims 1 or 2.

4. An expression vector comprising and expressing a nucleic acid molecule according to claim 3.

5. A host cell, comprising the nucleic acid molecule according to claim 3, or the vector according to claim 4.

6. An isolated T cell comprising a T-cell receptor (TCR), which specifically binds to a peptide according to claim 1 or 2.

7. A pharmaceutical composition, comprising a peptide according to claim 1 or 2, or a nucleic acid according to claim 3, a vector according to claim 4, a cell according to claim 5, an isolated T-cell according to claim 6.

8. The pharmaceutical composition according to claim 7, which is in the form of a vaccine

9. The peptide according to claim 1 or 2 for use in the prevention and/or treatment of a disease selected from transplant rejection, malignancies, .

10. The peptide for use according to claim 9, wherein the transplant rejection is selected from graft versus host disease or transplant rejections in the context of an allo-HSCT, and wherein said malignancies are selected from leukemias, lymphomas, and myelomas.

11. The peptide for use according to claim 9 or 10, wherein the cells of said malignancy express CYBA-72Y/B15.

12. The peptide for use according to any of claims 9 to 11, wherein said malignancy does not respond to prolonged treatment with chemotherapy, and/or is resistant to chemotherapy.

13. An *in vitro* method for generating T cells, comprising
a) providing a first cell that expresses a peptide according to claim 1 or 2,
b) bringing a population of peripheral blood mononuclear cells (PBMCs) into contact with said first cell, and thereby stimulating said PBMCs, and
c) selecting out of the population of stimulated PBMCs T cells which have the ability to recognize a cell expressing the peptide used in (a).

14. An *in vitro* method for diagnosing the suitability of a donor and/or recipient for an allo-HSCT, comprising the steps of:
a) determining the presence or absence of the mHAg according to SEQ ID No. 1 in a biological sample from a prospective donor and/or recipient of an HCST, and
b) identifying a suitable donor and/or recipient of an allo-HSCT based on, at least in part, said determining in a).

## Patentansprüche

1. Ein Peptid bestehend aus der Aminosäuresequenz gemäß SEQ ID Nr. 1 (GQK**Y**MTAVV), wobei genanntes Peptid die Fähigkeit aufweist, eine T-Zellantwort auszulösen.

2. Das Peptid gemäß Anspruch 1, wobei genanntes Peptid die Fähigkeit aufweist, an ein MHC Klasse I Molekül zu binden.

3. Ein isoliertes Nukleinsäuremolekül, welches für ein Peptid gemäß eines der Ansprüche 1 oder 2 kodiert.

4. Ein Expressionsvektor, der ein Nukleinsäuremolekül gemäß Anspruch 3 umfasst und exprimiert.

5. Eine Wirtszelle, umfassend das Nukleinsäuremolekül gemäß Anspruch 3, oder den Vektor gemäß Anspruch 4.

6. Eine isolierte T-Zelle umfassend einen T-Zellrezeptor (TCR), welcher spezifisch an ein Peptid gemäß der Ansprüche 1 oder 2 bindet.

7. Eine pharmazeutische Zusammensetzung, umfassend ein Peptid gemäß Anspruch 1 oder 2, oder eine Nukleinsäure gemäß Anspruch 3, oder einen Vektor gemäß Anspruch 4, eine Zelle gemäß Anspruch 5, eine isolierte T-Zelle gemäß Anspruch 6.

8. Die pharmazeutische Zusammensetzung gemäß Anspruch 7, welche in Form eines Impfstoffes vorliegt.

9. Das Peptid gemäß Anspruch 1 oder 2 zur Verwendung in der Vorbeugung und/oder Behandlung einer Erkrankung, die ausgesucht wird aus Transplantatzurückweisungen und bösartigen Krebserkrankungen.

10. Das Peptid zur Verwendung gemäß Anspruch 9, wobei die Transplantatzurückweisung ausgesucht wird aus Transplantat-Wirt-Reaktionen oder Transplantatzurückweisungen im Zusammenhang mit Fremd-HSCT und wobei die bösartigen Krebserkrankungen ausgesucht werden aus Leukämien, Lymphomen und Myelomen.

11. Das Peptid zur Verwendung gemäß Anspruch 9 oder 10, wobei die Zellen der genannten bösartigen Krebserkrankungen CYDA-72Y/B15 exprimieren.

12. Das Peptid zur Verwendung gemäß eines der Ansprüche 9-11, wobei genannter bösartiger Tumor nicht auf eine verlängerte Behandlung mit Chemotherapie reagiert, und/oder chemotherapieresistent ist.

13. Ein *In-vitro*-Verfahren zur Herstellung von T-Zellen, umfassend
a) Bereitstellen einer ersten Zelle, die ein Peptid gemäß der Ansprüche 1 oder 2 exprimiert,
b) In Kontakt bringen einer Population von peripheren blutmononukleären Zellen (PBMCs) mit der genannten ersten Zelle, und dadurch Stimulieren genannter PMBCs, und
c) Aussuchen von T-Zellen aus der Population der stimulierten PBMCs, welche die Fähigkeit aufweisen, eine Zelle, die das Peptid aus a) exprimiert, zu erkennen.

14. Ein *In-vitro*-Verfahren zum Diagnostizieren der Anwendbarkeit eines Spenders, und/oder Empfängers für eine Fremd-HSCT, umfassend die Schritte:
a) Feststellen der Anwesenheit oder Abwesenheit der mHAg gemäß SEQ ID Nr. 1 in einer biologischen Probe aus einem möglichen Spender und/oder Empfänger einer HCST, und
b) Identifizieren eines geeigneten Spenders und/oder Empfängers einer Fremd-HSCT basierend auf, zumindest teilweise, genanntem Feststellen in a).

## Revendications

1. Peptide constitué de la séquence d'acides aminés selon SEQ ID NO : 1 (GQK**Y**MTAVV), ledit peptide étant capable d'induire une réponse des lymphocytes T.

2. Peptide selon la revendication 1, ledit peptide étant capable de se lier à une molécule du CMH de classe I.

3. Molécule d'acide nucléique isolé, codant pour un peptide selon l'une quelconque des revendications 1 ou 2.

4. Vecteur d'expression comprenant et exprimant une molécule d'acide nucléique selon la revendication 3.

5. Cellule hôte, comprenant la molécule d'acide nucléique selon la revendication 3, ou le vecteur selon la revendication 4.

6. Lymphocyte T isolé comprenant un récepteur des lymphocytes T (TCR), qui se lie spécifiquement à un peptide selon la revendication 1 ou 2.

7. Composition pharmaceutique, comprenant un peptide selon la revendication 1 ou 2, ou un acide nucléique selon la revendication 3, un vecteur selon la revendication 4, une cellule selon la revendication 5, un lymphocyte T isolé selon la revendication 6.

8. Composition pharmaceutique selon la revendication 7, qui est sous la forme d'un vaccin.

9. Peptide selon la revendication 1 ou 2 pour une utilisation dans la prévention et/ou le traitement d'une maladie choisie parmi un rejet de transplant, des affections malignes.

10. Peptide pour une utilisation selon la revendication 9, le rejet de transplant étant choisi parmi la maladie du greffon contre l'hôte ou des rejets de transplants dans le contexte d'une allo-TCSH, et lesdites affections malignes étant choisies parmi des leucémies, des lymphomes, et des myélomes.

11. Peptide pour une utilisation selon la revendication 9 ou 10, les cellules de ladite affection maligne exprimant CYBA-72Y/B15.

12. Peptide pour une utilisation selon l'une quelconque des revendications 9 à 11, ladite affection maligne ne répondant pas à un traitement prolongé avec une chimiothérapie, et/ou étant résistante à une chimiothérapie.

13. Procédé *in vitro* pour la production de lymphocytes T, comprenant
a) la fourniture d'une première cellule qui exprime un peptide selon la revendication 1 ou 2,
b) la mise en contact d'une population de cellules mononucléées du sang périphérique (CMSP) avec ladite première cellule, et de cette façon la stimulation desdites CMSP, et
c) la sélection parmi la population des CMSP stimulées des lymphocytes T qui ont la capacité de reconnaître une cellule exprimant le peptide utilisé en (a).

14. Procédé *in vitro* pour diagnostiquer l'adéquation d'un donneur et/ou d'un receveur pour une allo-TCSH, comprenant les étapes suivantes :
a) la détermination de la présence ou de l'absence du mHAg selon SEQ ID NO : 1 dans un échantillon biologique provenant d'un donneur et/ou d'un receveur prospectif d'une TCSH, et
b) l'identification d'un donneur et/ou d'un receveur approprié d'une allo-TCSH en se basant, au moins en partie, sur ladite détermination en a).
